(19) Europäisches Patentamt
European Patent Office
Office européen des brevets

(11) EP 1 690 847 B1

(12) **EUROPEAN PATENT SPECIFICATION**

(45) Date of publication and mention of the grant of the patent:
**22.06.2016 Bulletin 2016/25**

(51) Int Cl.:
***C07C 13/567*** $^{(2006.01)}$ ***C07D 213/06*** $^{(2006.01)}$
***C09K 11/06*** $^{(2006.01)}$

(21) Application number: **06101717.4**

(22) Date of filing: **15.02.2006**

(54) **pi-conjugated compound having cardo structure, process for the preparation thereof and use thereof**

cardo-Struktur enthaltend pi-konjugierte Verbindungen, Verfahren zu ihrer Herstellung und deren Verwendung

Composés pi-conjugués ayant une structure de type cardo, procédé pour leur preparation et leur utilisation

(84) Designated Contracting States:
**DE GB NL**

(30) Priority: **15.02.2005 JP 2005038241**

(43) Date of publication of application:
**16.08.2006 Bulletin 2006/33**

(73) Proprietor: **TOSOH CORPORATION**
**Shunan-shi**
**Yamaguchi-ken 746-8501 (JP)**

(72) Inventors:
- **Nishiyama, Masakazu**
**Shunan-shi, Yamaguchi 746-0011 (JP)**
- **Matsumoto, Naoki**
**Shunan-shi, Yamaguchi 746-0024 (JP)**
- **Miyazaki, Takanori**
**Shunan-shi, Yamaguchi 746-0082 (JP)**
- **Eguchi, Hisao**
**Shunan-shi, Yamaguchi 746-0052 (JP)**

(74) Representative: **TBK**
**Bavariaring 4-6**
**80336 München (DE)**

(56) References cited:
**EP-A- 1 400 578 US-A1- 2002 132 134**

- **DATABASE CA [Online] CHEMICAL ABSTRACTS SERVICE, COLUMBUS, OHIO, US; NISHIYAMA, MASAKAZU ET AL: "Preparation and application of amine compounds having fluorene group as framework" XP002385839 retrieved from STN Database accession no. 2005:673249 & WO 2005/068413 A1 (TOSOH CORPORATION, JAPAN) 28 July 2005 (2005-07-28)**
- **"RÖMPP CHEMIE LEXICON", 1989, J. Falbe, M. Regitz pages 154-154,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 690 847 B1

**Description**

Technical Field

This invention relates to a π-conjugated compound having a cardo structure, a process for preparing the π-conjugated compound, and an organic electroluminescence (EL) element.

The π-conjugated compound having a cardo structure can be widely used as an organic semiconductor material. More particularly it can be used as a light emitting material or an electron transport material of an organic EL element used for a flat light source or a display, or as an organic transistor material.

Background Art

In recent years, a wide spread attention is attracted to an electroluminescence (EL) element for flat panel display characterized as autogeneous light emission, high-speed response and wide view angle. As a material for the EL element, an increasing attention is paid to an organic light emitting material. The first benefit of an organic light emitting material lies in the fact that the optical characteristics can be desirably varied to a certain extent depending upon the designed molecular structure. Thus, a full-color organic light emitting element is available by using light emitting materials of red, green and blue primary colors.

Heretofore, many organic light emitting materials have been developed. However, most of the organic light emitting materials have a low utility because of a low solubility in organic solvents and a high crystallizability. For examples, polyphenylene compounds as represented by sexiphenyl derivatives and analogues have a high crystallizability, and therefore, when a thin film is formed from these compounds by vacuum deposition, cohesion tends to occur and a stable film is difficult to obtain. When thin films made from these compounds are used for an organic thin-film device such as an organic EL element, dark spots and short-circuit points tend to occur (see, for example, Japanese Unexamined Patent Publication No. H7-278537 and Organic EL Material and Display (Japanese publication), page 195, published by CMC Publishing Co., Ltd., Japan).

It is described in Japanese Unexamined Patent Publication No. H7-278537 that spiro-6ϕ prepared by bonding sexiphenyl with spiro quaternary carbon has a high glass transition temperature (Tg), and is not crystallized over a long period of time as compared with other organic EL elements made by a spin coating method, and spiro-6ϕ exhibits blue color light emission. However, these benefits still do not reach the desired level.

Bathophenanthroline used as an electron transport material and a hole blocking material exhibits a high electron mobility, but its thin film has poor stability and its EL element has poor durability. To enhance durability of thin films made from phenanthroline derivatives and electron affinity of these derivatives, phenanthroline derivatives having introduced therein a 9,9-dialkylfluorenylene group have been proposed in Japanese Unexamined Patent Publication No. 2004-277377.

WO2005068413 (A1), which is a prior art document pursuant to Article 54(3) EPC, discloses novel amine compounds utilizable as a hole-transporting or hole-injecting material in organic electroluminescent elements, electrophotographic photoreceptors, etc. The novel amine compound is represented by the following general formula (1) of WO2005068413 (A1).

EP1400578 (A1) discloses arylamine derivatives that can be utilized as hole transport or hole injection materials of organic electroluminescence devices, electrophotographic reactors, etc., and synthetic intermediates thereof, and processes of producing those. The arylamine derivative is represented by the general formula (1) of EP1400578.

US2002132134 (A1) mentions an electroluminescent device containing an anode, an organic electroluminescent element, and a cathode wherein the electroluminescent element contains, for example, a fluorescent hydrocarbon component of Formula (I) wherein R1 and R2 are substituents, which are selected from the group consisting of hydrogen, an alkyl, an alicyclic alkyl, an alkoxy, a halogen, and a cyano; Ar1 and Ar2 are each independently an aromatic component or an aryl group comprised of a from about 4 to about 15 conjugate-bonded or fused benzene rings.

Disclosure of the Invention

A primary object of the present invention is to provide a light emitting material, especially light emitting material exhibiting blue color, a hole blocking material and an electron transport material, giving a stable thin film which is not crystallized over a long period of time, and has enhanced durability, and can be made by spin coating due to its high solubility, as well as by vacuum deposition.

The inventors made extensive researches and found that (i) specific π-conjugated compounds having a cardo structure have a high glass transition temperature which can be an indicator showing a high heat stability; (ii) most of the π-conjugated compounds are not crystalline and have an amorphous structure, and thus give a thin film of enhanced stability; (iii) even though a part of the π-conjugated compounds is crystalline, a thin film made from the π-conjugated

compounds even by a coating method such as spin coating as well as a vacuum deposition method does not become white, turbid over a long period of time, which would be due to the fact that the π-conjugated compounds have a cardo structure (note, the term "cardo" refers to a hinge and thus a structure such that cyclic groups are bonded directly to the back bone chain) ; and (iv) the π-conjugated compounds having a cardo structure are especially suitable as a light emitting material, a hole blocking material and an electron transport material in an organic electroluminescence element. The present invention has been completed on the basis of these findings.

Thus, in one aspect of the present invention, there is provided a π-conjugated compound as defined in claim 1.

In another aspect of the present invention, there is provided a process for preparing the above-mentioned π -conjugated compound as defined in claim 2.

In a further aspect of the present invention, there is provided an organic electroluminescence element characterized as having a light emitting layer, a hole blocking layer or an electron transport layer, which layers comprise the above-mentioned π-conjugated compound.

Brief Description of the Drawings

Fig. 1 shows evaluation results of reversibility of peak by cyclic voltammetry on an EL element with a π-conjugated compound.
Fig. 2 shows evaluation results of reversibility of peak by cyclic voltammetry on an EL element with bathophenanthroline.
Fig. 3 shows a relationship of current density with measurement voltage.
Fig. 4 shows a relationship of luminance with measurement voltage.

Description of the Preferred Embodiments

In formula (1) representing the π-conjugated compound having a cardo structure, $Ar^1$ independently represents a group represented by the following formula (3):

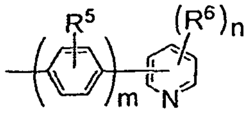

(3)

where $R^5$ and $R^6$ independently represent a hydrogen atom, a straight-chain, branched or cyclic alkyl or alkoxy group having 1 to 18 carbon atoms, and m is an integer of 0
to 3, and n is an integer of 0 to 2.

As specific examples of the straight-chain, branched or cyclic alkyl group having 1 to 18 carbon atoms which may have a substituent, in formulae (1), there can be mentioned a methyl group, an ethyl group, a propyl group, an isopropyl group, a n-butyl group, a sec-butyl group, a tert-butyl group, a pentyl group, a hexyl group, a heptyl group, an octyl group, a stearyl group, a trichloromethyl group, a trifluoromethyl group, a cyclopropyl group, a cyclohexyl group, a 1,3-cyclohexadienyl group and a 2-cyclopenten-1-yl group.

As specific examples of the straight-chain, branched or cyclic alkoxy group having 1 to 18 carbon atoms which may have a substituent, in formulae (1), there can be mentioned a methoxy group, an ethoxy group, a propoxy group, an isopropoxy group, an n-butoxy group, a sec-butoxy group, a tert-butoxy group, a pentyloxy group, a hexyloxy group, a stearyloxy group and a trifluoromethoxy group.

As specific examples of an aryloxy group having 6 to 24 carbon atoms ther can be mentioned a phenoxy group.

As specific examples of an halogen atom,

there can be mentioned fluorine, chlorine, bromine and iodine atoms.

In formula (1) representing the pi-conjugated compound having a cardo structure, $Ar^2$ is as defined in claim 1.

As specific examples of an aryl group, there can be mentioned a phenyl group, a 1-naphthyl group, a 2-naphthyl group, a 2-anthryl group, a 9-anthryl group, a 2-fluorenyl group, a phenanthryl group, a pyrenyl group, a chrysenyl group, a perylenyl group and a picenyl group. A heteroaryl group includes, for example, those which are an aromatic group having at least one hetero atom selected from an oxygen atom, a nitrogen atom and a sulfur atom, and, as specific examples thereof, there can be mentioned 4-quinolyl group, 4-pyridyl group, 3-pyridyl group, 2-pyridyl group, 2-dipyridyl group, 1,10-phenanthrolinyl group, 3-furyl group, 2-furyl group, 3-thienyl group, 2-thienyl group, 2-oxazolyl group, 2-thiazolyl group, 2-benzoxazolyl group, 2-benzothiazolyl group and 2-benzoimidazolyl group.

Of the above-recited aryl and heteroaryl groups, preferable are fused-ring aromatic groups such as a naphthyl group, a phenanthryl group, a fluorenyl group, an anthryl group, a pyrenyl group, a chrysenyl group, a picenyl group, a perylenyl group and a benzo[c]fluorenyl group; heteroaryl groups such as 1,10-phenanthrolinyl group; and those which are represented by the following formula (6a) or (6b):

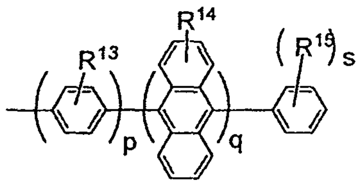

(6a)

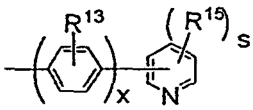

(6b)

where $R^{13}$, $R^{14}$ and $R^{15}$ independently represent a hydrogen atom, a straight-chain, branched or cyclic alkyl or alkoxy group having 1 to 10 carbon atoms, or an aryl group having 6 to 24 carbon atoms, or a heteroaryl group having 3 to 24 carbon atoms ; and p and q are integers satisfying the following formula:

$$1 \leqq (p + q) \leqq 3$$

and s and x are integers of 0 to 2.

Of the above-recited aryl and heteroaryl groups, more preferable are fused-ring aromatic groups and hetero aryl groups, which are represented by the following formulae (4a), (4b) and (5).

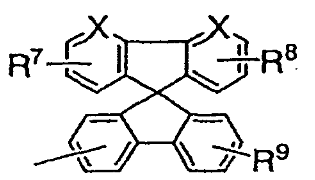

(4a)

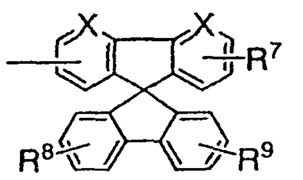

(4b)

where $R^7$, $R^8$ and $R^9$ independently represent a hydrogen atom, a straight-chain, branched or cyclic alkyl or alkoxy group having 1 to 18 carbon atoms, or an aryl or aryloxy group having 6 to 24 carbon atoms, or a heteroaryl group having 3 to 24 carbon atoms, or a halogen atom; and X represents a carbon atom or a nitrogen atom.

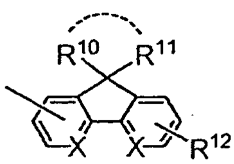

(5)

where $R^{10}$, $R^{11}$ and $R^{12}$ independently represent a hydrogen atom, a straight-chain, branched or cyclic alkyl or alkoxy group having 1 to 18 carbon atoms, or an aryl or aryloxy group having 6 to 24 carbon atoms, or a heteroaryl group having 3 to 24 carbon atoms, or a halogen atom, provided that $R^{10}$ and $R^{11}$ may be bonded together with each other to form a ring; and X represents a carbon atom or a nitrogen atom.

As specific examples of the alkyl, alkoxy, aryl, heteroaryl and aryloxy groups in formulae (4a), (4b), (5), (6a) and (6b), there can be mentioned those which are recited for the alkyl, alkoxy, aryl, hetero-aryl and aryloxy groups in formulae (1), (2) and (3).

Specific examples and reference examples of the π-conjugated compound having a cardo structure of formula

(1) are recited in the following Tables 1 through 5, that by no means limit the scope of the invention.

Table 1

| Compound | $Ar^1$ | $Ar^2$ | $R^1$ | $R^2$ |
|---|---|---|---|---|
| 1* | 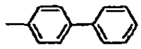 | 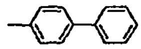 | H | H |
| 2* | 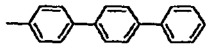 | 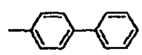 | H | H |
| 3* | 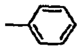 | 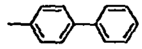 | H | H |
| 4* | 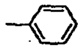 | 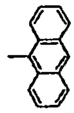 | H | H |
| 5* | 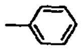 | 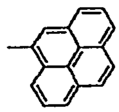 | H | H |
| 6* | 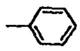 | 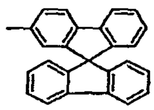 | H | H |
| 7* | 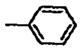 | 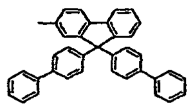 | H | H |
| 8* | 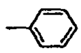 | 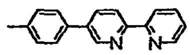 | H | H |
| 9* | 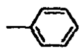 | 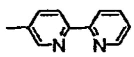 | H | H |
| 10* | 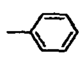 | 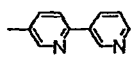 | H | H |
| 11* | 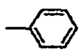 | 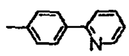 | H | H |
| 12* | 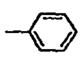 | 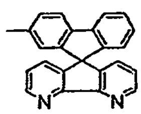 | H | H |
| 13* | 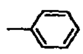 | 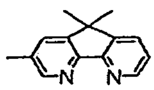 | H | H |

(continued)

| Compound | Ar¹ | Ar² | R¹ | R² |
|---|---|---|---|---|
| 14* | 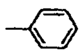 | 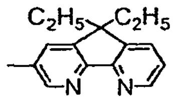 | H | H |
| * reference exmaple | | | | |

Table 2

| Compound | Ar¹ | Ar² | R¹ | R² |
|---|---|---|---|---|
| 15* | 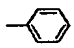 | 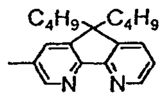 | H | H |
| 16* | 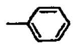 | 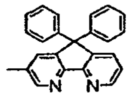 | H | H |
| 17* | 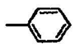 | 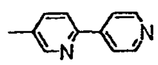 | H | H |
| 18* | 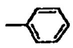 | 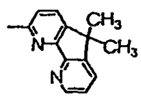 | H | H |
| 19* | 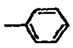 | 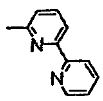 | H | H |
| 20* | 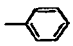 | 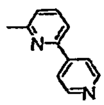 | H | H |
| 21* | 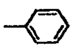 | 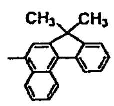 | H | H |
| 22* | 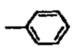 | 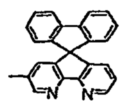 | H | H |
| 23* | 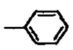 | 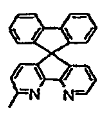 | H | H |
| 24 | 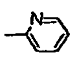 | 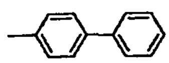 | H | H |

(continued)

| Compound | $Ar^1$ | $Ar^2$ | $R^1$ | $R^2$ |
|---|---|---|---|---|
| 25 | 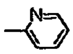 | 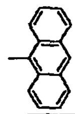 | H | H |
| 26 | 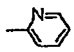 | 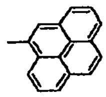 | H | H |
| 27 | 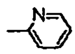 | 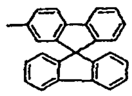 | H | H |
| * reference example | | | | |

Table 3

| Compound | $Ar^1$ | $Ar^2$ | $R^1$ | $R^2$ |
|---|---|---|---|---|
| 28 | 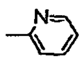 | 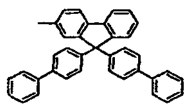 | H | H |
| 29 | 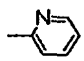 | 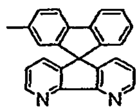 | H | H |
| 30 | 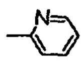 | 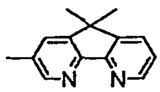 | H | H |
| 31 | 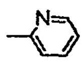 | 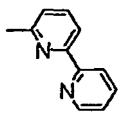 | H | H |
| 32 | 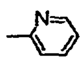 | 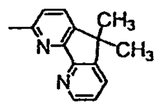 | H | H |
| * 33* | 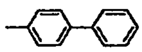 | 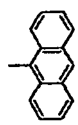 | H | H |
| 34 | 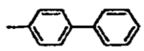 | 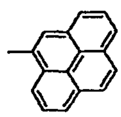 | H | H |

(continued)

| Compound | $Ar^1$ | $Ar^2$ | $R^1$ | $R^2$ |
|---|---|---|---|---|
| 35* | 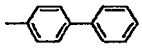 | 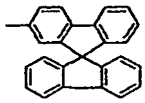 | H | H |
| * 36 | 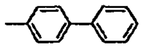 | 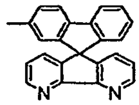 | H | H |
| 37* | 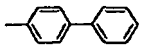 | 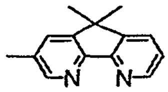 | H | H |
| * reference compound | | | | |

Table 4

| Compound | $Ar^1$ | $Ar^2$ | $R^1$ | $R^2$ |
|---|---|---|---|---|
| 38* | 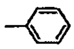 | 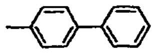 | 3- $CH_3$ | 3- $CH_3$ |
| 39* | 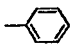 | 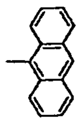 | 3- $CH_3$ | 3- $CH_3$ |
| 40* | 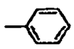 | 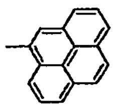 | 3- $CH_3$ | 3- $CH_3$ |
| 41* | 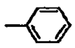 | 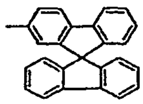 | 3- $CH_3$ | 3-$CH_3$ |
| 42* | 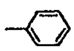 | 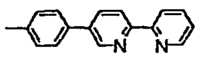 | 3- $CH_3$ | 3- $CH_3$ |
| 43* | 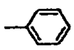 | 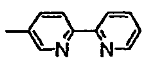 | 3- $CH_3$ | 3- $CH_3$ |
| 44* | 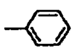 | 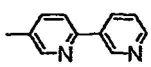 | 3- $CH_3$ | 3- $CH_3$ |
| 45* | 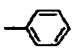 | 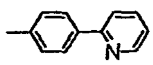 | 3- $CH_3$ | 3- $CH_3$ |

(continued)

| Compound | $Ar^1$ | $Ar^2$ | $R^1$ | $R^2$ |
|---|---|---|---|---|
| 46* | 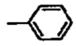 | 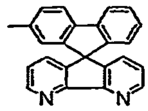 | 3-$CH_3$ | 3- $CH_3$ |
| 47* | 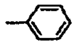 | 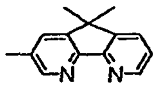 | 3- $CH_3$ | 3- $CH_3$ |
| 48* | 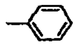 | 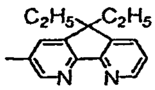 | 3- $CH_3$ | 3- $CH_3$ |
| 49* | 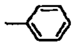 | 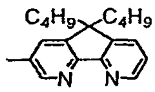 | 3- $CH_3$ | 3- $CH_3$ |
| 50* | 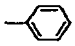 | 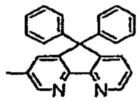 | 3- $CH_3$ | 3-$CH_3$ |
| 51* | 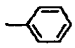 | 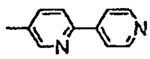 | 3- $CH_3$ | 3- $CH_3$ |
| * reference compound | | | | |

Table 5

| Compound | $Ar^1$ | $Ar^2$ | $R^1$ | $R^2$ |
|---|---|---|---|---|
| 52* | 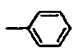 | 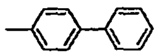 | 3-phenyl | 3-phenyl |
| * 53* | 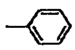 | 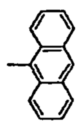 | 3-phenyl | 3-phenyl |
| 54* | 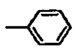 | 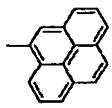 | 3-phenyl | 3-phenyl |
| 55* | 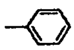 | 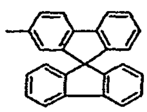 | 3-phenyl | 3-phenyl |
| 56* | 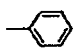 | 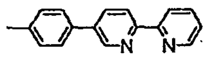 | 3-phenyl | 3-phenyl |

(continued)

| Compound | $Ar^1$ | $Ar^2$ | $R^1$ | $R^2$ |
|---|---|---|---|---|
| 57* | 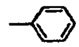 | 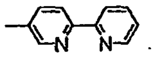 | 3-phenyl | 3-phenyl |
| 58* | 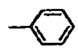 | 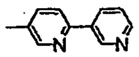 | 3-phenyl | 3-phenyl |
| 59* | 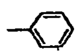 | 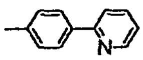 | 3-phenyl | 3-phenyl |
| 60* | 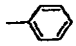 | 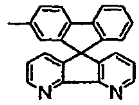 | 3-phenyl | 3-phenyl |
| 61* | 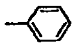 | 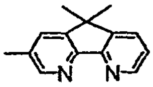 | 3-phenyl | 3-phenyl |
| 62* | 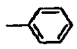 | 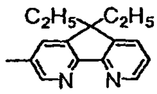 | 3-phenyl | 3-phenyl |
| 63* | 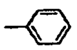 | 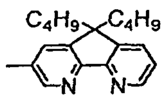 | 3-phenyl | 3-phenyl |
| 64* | 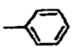 | 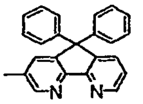 | 3-phenyl | 3-phenyl |
| 65* | 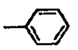 | 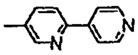 | 3-phenyl | 3-phenyl |
| * reference compound | | | | |

The π-conjugated compound of formula (1) can be synthesized according to a known type of reaction scheme (For example, see Chem. Rev. 19955, 95, p2457-2483). A typical example of the synthesis process comprises the steps of (i) allowing a fluorene intermediate compound represented by the formula (7), shown below, to react with a boric acid compound A represented by the formula (8), shown below, in the presence of a transition metal catalyst; (ii) deblocking the thus-obtained reaction product in the presence of an acid catalyst; (iii) trifluoromethanesulfonylating the deblocked product; and then (iv) allowing the trifluoromethanesulfonylated product to react with a boric acid compound B represented by the formula (9), shown below, in the presence of a transition metal catalyst.

The transition metal catalyst as used in step (i) includes, for example, a nickel catalyst and a palladium catalyst, and, as specific examples thereof, there can be mentioned nickel catalysts such as 1,4-bis(diphenylphosphino)butanenickel(II) chloride, 1,1'-bis(diphenylphosphino)ferrocenenickel(II) chloride, 1,2-bis(diphenylphosphino)ethanenickel(II) chloride and 1,3-bis(diphenylphosphino)propanenickel(II) chloride; and palladium catalysts such as tetrakis(triphenylphosphine)-palladium(0), bis(triphenylphosphino)palladium(II) chloride, 1,1'-bis(diphenylphosphino)ferrocenepalladium(II) chloride, 1,2-bis(diphenylphosphino)ethanepalladium(II) chloride, 1,3-bis(diphenylphosphino)propanepalladium(II) chloride, 1,4-bis(diphenylphosphino)butanepalladium(II) chloride, bis(tri-tert-butylphosphine)palladium(0), a polymer- fixed palladium catalyst and palladium carbon.

Formula (7):

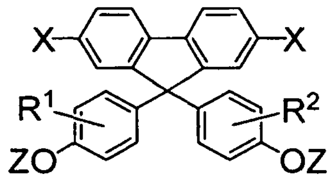

(7)

where Z is a protecting group for a phenol group, $R^1$ and $R^2$ independently represent a hydrogen atom, a straight-chain, branched or cyclic alkyl or alkoxy group having 1 to 18 carbon atoms which may have a substituent, a phenyl, naphthyl or phenoxy group which may have a substituent, or a halogen atom, and X represents an iodine atom, a bromine atom or a chlorine atom; Formulae (8) and (9):

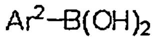

or

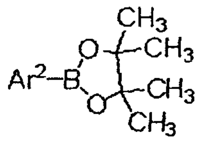

(8)

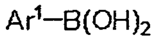

or

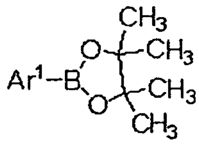

(9)

where, $Ar^2$ in formula (8) is as defined in claim 3;

and $Ar^1$ in formula (9) independently represents a group represented by the following formula (3):

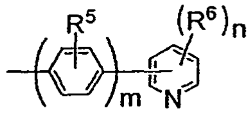

(3)

where $R^5$ and $R^6$ independently represent a hydrogen atom, a straight-chain, branched or cyclic alkyl or alkoxy group having 1 to 18 carbon atoms and m is an integer

of 0 to 3, and n is an integer of 0 to 2.

The protecting group Z in formula (7) is not particularly limited and can be chosen from known protecting groups for phenolic hydroxyl groups (see, for example, Protecting Group in Organic Synthesis, published by John Wiley & Sons). In view of ease in deblocking, alkoxyalkyl groups such as ethoxyethoxymethyl group and methoxymethyl group are preferable.

The protecting group Z for phenolic hydroxyl group can be released in the presence of an acid catalyst. The acid catalyst includes, for example, hydrochloric acid, nitric acid, sulfuric acid, p-toluenesulfonic acid, methanesulfonic acid and boron trifluoride. Of these acid catalysts, hydrochloric acid is preferable.

A typical specific example of the process for synthesizing the π-conjugated compound wherein Z is a methoxymethyl group is schematically shown in the following reaction scheme.

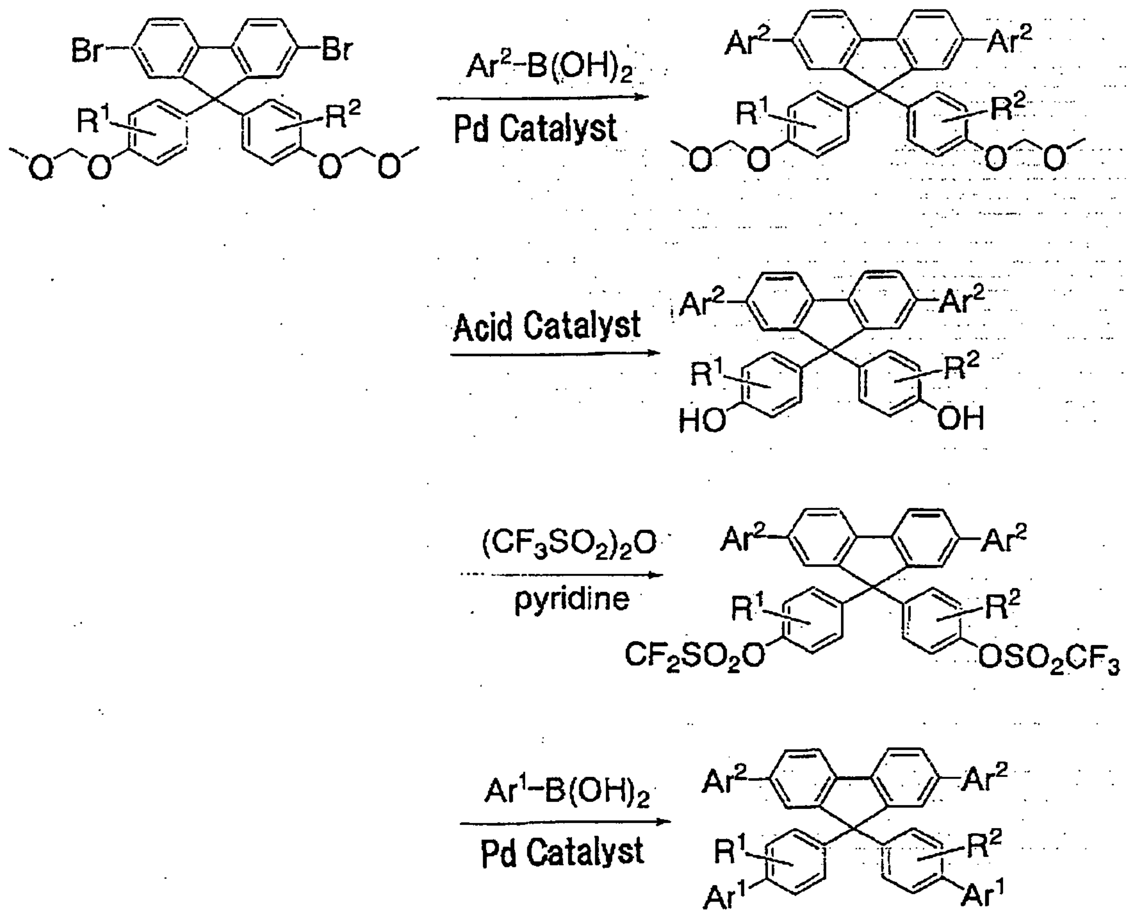


The π-conjugated compound having a cardo structure according to the present invention can be used as a blue-color light-emitting material, a hole blocking material and an electron transport material in an organic electroluminescence element. The hole blocking material preferably has an ionizing potential larger than those of light-emitting materials used in the element, which include, for example, aluminum trisquinolinol complex ($Alq_3$) as a green fluorescent material, and 4,4'-N,N'-dicarbazole-biphenyl (CBP) as a phosphorescence host material. The electron transport material preferably has an electron affinity equal to or larger than those of conventional electron transport materials such as $Alq_3$ and bathophenanthroline. Further, the electron transport material preferably has an electron mobility larger than those (approximately $10^{-6}$ $cm^2/V.s$) of conventional electron transport materials such as $Alq_3$. The ionizing potential can be measured generally by photoelectron spectroscopy or cyclic voltammetry. The electron affinity can be determined from the ionizing potential as measured by photoelectron spectroscopy, and from the energy at an absorption spectrum end, or measured by cyclic voltammetry.

The π-conjugated compound having a cardo structure according to the present invention gives a thin film having far enhanced stability as compared with the conventional thin films. Therefore, the π-conjugated compound can be used as an organic transistor material, and an organic semiconductor material in a photoelectric transfer element, a solar battery and an image sensor, as well as a light-emitting material, a hole blocking material and an electron transport material in an organic EL element and an electrophotography photoconductor.

Examples

The invention will be specifically described by the following examples.

In the examples, % is by weight unless otherwise specified.

Field desorption mass spectroscopy (FDMS) measurement was carried out using M-80B available from Hitachi Ltd.

NMR measurement was carried out using GEMINI-200 available from Varian Technologies Japan Ltd.

Gas chromatography mass spectrometry (GCMS) measurement was carried out using JMS-K9 available from JEOL Ltd.

Elemental analysis measurement was carried out using 2400II available from Perkin-Elmer Japan Co., Ltd.

Synthesis Example 1

(Synthesis of 2,7-dibromo-9,9'-bis[4-(methoxymethyloxy)phenyl]-9H-fluorene)

A 100 mL Kjeldahl flask was charged with 0.82 g (34.2 mmol) of sodium hydride and 25 mL of tetrahydrofuran under a nitrogen gas stream, and the content was cooled to 0°C. To the cooled content, a solution in tetrahydrofuran of 6.5 g (14.3 mmol) of 2, 7-dibromo-4, 4' - (9-fluorenylidene) diphenol was dropwise added, and then, 3.44 g (42.7 mmol) of chloromethyl methyl ether was dropwise added. Then the reaction mixture was stirred at room temperature for 12 hours, and 10 mL of methanol was added to decompose sodium hydride. 20 mL of toluene was added to separate an organic phase. The organic phase was washed with water and then with an aqueous saturated sodium chloride solution, and then the organic phase was concentrated. The concentrate was recrystallized from ethanol to isolate 6.7 g (yield: 80%) of 2,7-dibromo-9,9'-bis[4-(methoxymethyloxy)phenyl]-9H-fluorene.

Synthesis Example 2

(Synthesis of 2,7-dibromo-9,9-bis(biphenylyl)fluorene)

0.2 L of a solution in tetrahydrofuran of 3.3 g (135 mmol) of magnesium and 31. 4 g (135 mmol) of 2-bromobiphenyl was prepared. While the solution was maintained at 40°C, the solution was dropwise added into a 1L separable flask having charged with a mixed solution of 35 g (104 mmol) of 2,7-dibromofluorenone and 280 mL of tetrahydrofuran. After completion of the addition, the reaction liquid was stirred at 40°C for 3 hours. After completion of the reaction, 420 g of an aqueous 10% ammonium chloride solution was added at room temperature to the reaction liquid, and then an organic phase was separated. The organic phase was dried over anhydrous magnesium sulfate, filtered and then concentrated. The concentrate was recrystallized from toluene to give 36 g of a carbinol compound.

A 1L separable flask was charged with 34 g of the carbinol compound, 107 g of biphenyl, acetic acid 480 g and 27 g of sulfuric acid, and the mixture was stirred at 80°C for the night. The reaction liquid was allowed to cool to room temperature, and then placed in ice-water, while being stirred, to terminate the reaction. The thus-obtained precipitate was washed with water and then with hot ethanol. Finally recrystallization from toluene gave 27 g of 2,7-dibromo-9,9-bis(biphenylyl)fluorene (melting point: 277-282°C, yield: 80%).

The identification of 2,7-dibromo-9,9-bis(biphenylyl)-fluorene was conducted by FDMS, $^{1}$H-NMR and $^{13}$C-NMR.
FDMS: 628
$^{1}$H-NMR: ($CDCl_3$, ppm) ; 7.23-7.64 (m, 24H)
$^{13}$C-NMR: ($CDCl_3$, ppm); 152.8, 143.2, 140.4, 140.0, 138.0, 131.0, 129.4, 128.7, 128.3, 127.3, 127.2, 126.9, 121.9, 121.6, 65.2

Synthesis Example 3

(Synthesis of 2-bromo-9,9-bis(biphenylyl)fluorene)

The procedure as described in Synthesis Example 2 was repeated wherein 2-bromofluorenone was used instead of 2,7-dibromofluorenone with all other conditions remaining substantially the same. Thus, 2-bromo-9,9-bis(biphenylyl)-fluorene was obtained.

The identification of 2-bromo-9,9-bis(biphenylyl)-fluorene was conducted by FDMS.
FDMS: 548

Synthesis Example 4

(Synthesis of 2-bromo-9,9'-spirobifluorene)

A 500 mL Kjeldahl flask was charged with 15 g (40.8 mmol) of 2-bromofluorene, 329 mg (2.5 mol%) of tetrabutylammonium bromide, 3.73 g of an aqueous 48% sodium hydroxide solution and 220 mL of toluene. The content was stirred for the night while the content was maintained at 60°C and air was blown therein. The reaction liquid was concentrated, and 80 mL of water was added to the concentrate. The thus-obtained precipitate was filtered and then washed until the pH value became neutral. Recrystallization of the washed precipitate from a mixed liquid of ethanol/tetrahydrofuran gave 10.5 g of 2-bromofluorenone (yellow needle crystal; melting point: 145-147°C).

The identification of 2-bromofluorenone was conducted by $^{1}$H-NMR and $^{13}$C-NMR.
$^{1}$H-NMR: ($CDCl_3$, ppm); 7.30-7.76 (m, 7H)
$^{13}$C-NMR: ($CDCl_3$, ppm); 192.32, 143.68, 143.00, 137.11, 135.79, 135.04, 133.72, 129.45, 127.59, 124.64, 122, 95,

121.74, 120.46

A 300 mL Kjeldahl flask was charged with a Grignard solution prepared from 1.0 g (42.2 mmol) of magnesium and 9.85 g (42.2 mmol) of 2-bromobiphenyl. To the Grignard solution, a solution of 9.9 g of 2-bromofluorenone in 130 mL of tetrahydrofuran was dropwise added at room temperature. The mixture was heated under reflux for 14 hours. After the completion of reaction, the reaction liquid was separated. An organic phase was washed with an aqueous 10% ammonium chloride solution, and then concentrated. Recrystallization of the concentrate from toluene gave 8.8 g of 2-bromo-9-(2-biphenylyl)-9-hydroxyfluorene. The obtained 2-bromo-9- (2-biphenylyl) -9-hydroxyfluorene was mixed with 65 mL of acetic acid, and the mixture was heated to 100°C. Several drops of a concentrated aqueous hydrochloric acid solution was added to the heated mixture, and the mixture was maintained at that temperature for 1.5 hours while being stirred. After the completion of reaction, the reaction liquid was incorporated in 120 g of ice-water. The thus-obtained precipitate was filtered and washed with water. Recrystallization of the precipitate from a chloroform/ethanol mixed liquid gave 8.1 g of 2-bromo-9,9'-spirobifluorene (melting point: 181-183°C).

The identification of 2-bromo-9,9'-spirobifluorene was conducted by FDMS and $^{13}$C-NMR.

FDMS: 394

$^{13}$C-NMR: ($CDCl_3$, ppm); 150.75, 148.48, 147.83, 141.66, 140.71, 140.58, 130.84, 128.20, 127.93, 127.89, 127.23, 124.05, 124.01, 121.36, 121.26, 120.09, 120.00, 65.85

Synthesis Example 5

(Synthesis of 9,9-bis(biphenylyl)fluorene-2,7-bis(4,4,5,5-tetramethyl-[1,3,2]dioxaborolane)

A 100 mL Kjeldahl flask was charged with 1.68 mmol of 2,7-dibromo-9,9-bis(biphenylyl)fluorene, prepared in Synthesis Example 2, 3.77 mmol of bis(pinacolate)diborane, 10.1 mmol of potassium acetate, 0.034 mmol of bis(diphenylphosphino)-ferrocene dichloropalladium and 15 mL of anhydrous dimethylformamide. The reaction mixture was maintained at 80°C for 2 hours while being stirred. After the completion of reaction, 20 mL of toluene and 20 mL of water were added to extract the reaction product. An organic phase was washed with an aqueous saturated sodium chloride solution and then with water, and was then concentrated to give a powder. The powder was recrystallized from a tetrahydrofuran/ethanol mixed liquid to 0.61 g of the desired compound.

The identification of the compound was conducted by FDMS, $^{1}$H-NMR and $^{13}$C-NMR.

FDMS: 721

$^{1}$H-NMR: ($CDCl_3$, ppm); 1.31 (s, 24H), 7.26-7.58 (m, 18H), 7.84-7.88 (m, 6H)

$^{13}$C-NMR: ($CDCl_3$, ppm); 25.03, 65.10, 83.82, 119.98, 126.96, 127.09, 128.68, 128.85, 132,34, 134.39, 139.28, 140.78, 142.84, 144.69, 151.04

Synthesis Example 6

(Synthesis of 4,5-diaza-2-bromo-9,9'-spirobifluorene)

A Grignard reagent was prepared from 0.40 g (16.3 mmol) of magnesium, 3.82 g (16.4 mmol) of 2-bromobiphenyl and 20 mL of tetrahydrofuran. A solution of 2.71 g (14.9 mmol) of 4,5-diazafluorenone in 65 mL of tetrahydrofuran was dropwise added to the Grignard reagent under reflux, and heated under reflux for 21 hours. Then the reaction liquid was allowed to cool to room temperature, and 50 g of water was added to terminate the reaction. The reaction liquid was extracted with two 50 mL portions of chloroform, and then dried over anhydrous magnesium sulfate. The extracts were filtered, concentrated and then washed with hexane to give 3.75 g of a light brown powder. A 200 mL Kjeldahl flask was charged with the thus-obtained powder and 75 mL of acetic acid, and the content was heated to 100°C. To the content, 4.97 g of concentrated sulfuric acid was added and the mixture was stirred under heated conditions for 22 hours. The reaction liquid was incorporated in 50 g of cold water to terminate the reaction. An aqueous 30% sodium hydroxide solution was added to the reaction liquid to adjust the pH value to 11. Then the reaction liquid was extracted with two 100 mL portions of chloroform, and dried over anhydrous magnesium sulfate. The extracts were filtered, and concentrated. Recrystallization of the concentrate from a hexane/ethanol mixed liquid gave 1.69 g of 4,5-diaza- 9,9'-spirobifluorene (yield: 48%, melting point: 208-210°C).

The identification of 4, 5-diaza- 9,9'-spirobifluorene was conducted by FDMS, $^{1}$H-NMR and $^{13}$C-NMR.

FDMS: 318

$^{1}$H-NMR: ($CDCl_3$, ppm); 8.72-8.76 (m, 2H), 7.87 (d, J=7.2Hz, 2H), 7.33-7.41 (m, 2H), 7.11-7.18 (m, 6H), 6.73 (d, J=7.2Hz, 2H)

$^{13}$C-NMR: ($CDCl_3$, ppm); 158.3, 149.8, 145.6, 143.1, 141.4, 131.4, 128.0, 127.7, 123.5, 123.3, 120.0, 61.8

A 50 mL Kjeldahl flask was charged with 0.5 g (1.57 mmol) of 4,5-diaza-9,9'-spirobifluorene, 0.25 g (1.56 mmol) of iron chloride and 5 mL of dichloromethane. 0.25 g (1.56 mmol) of bromine was dropwise added to the content in an

ice-water bath, and then, the reaction liquid was stirred at room temperature over the night. An aqueous saturated sodium hydrogen carbonate solution was added to the reaction liquid to terminate the reaction. Then the reaction liquid was extracted with two 15 mL portions of chloroform, dried over magnesium sulfate, and then concentrated. Recrystallization of the concentrate from toluene gave 0.11 g of 4,5-diaza-2-bromo-9,9'-spirobifluorene (yield: 18%).

Synthesis Example 7

(Synthesis of 2-bromo-9,9-dimethyl-4,5-diazafluorene)

A 2L separable flask was charged with 18 g (99.9 mmol) of fenanthroline, 18 g (320.8 mmol) of potassium hydroxide and 900 g of water, and the content was heated to 80°C. A mixed liquid comprising 45 g of potassium permanganate and 720 g of water was dropwise added to the content while the content was stirred at that temperature. After the completion of addition, the stirring was further continued for 30 minutes and the reaction was terminated. The hot reaction liquid was filtered to remove manganese dioxide. Then the reaction liquid was allowed to cool to room temperature, and then extracted with chloroform. The extract was treated in the conventional manner, and concentrated to give a yellow powder. Recrystallization of the powder from acetone gave 7.54 g of a yellow needle crystal (yield: 41%).

The identification of the crystalline compound was conducted by $^1$H-NMR and $^{13}$C-NMR.

$^1$H-NMR: ($CDCl_3$, ppm); 8.80 (d, J=5.2, 2H), 8.05 (dd, J=7.6, 1.6, 2H), 7.36 (dd, J=7.6, 5.2, 2H)

$^{13}$C-NMR: ($CDCl_3$, ppm); 189.42, 163.31, 155.10, 131.45, 129.30, 124.69

A 300 mL Kjeldahl flask was charged with 7.5 g (41.4 mmol) of the thus-obtained 4,5-diazafluorenone, 7.5 g (187.5 mmol) of sodium hydroxide, 5.3 g (165.4 mmol) of 98% hydrazine, and 130 mL of diethylene glycol, and the content was maintained at 160°C for 18 hours while being stirred. After the completion of reaction, 250 mL of water was added to the reaction liquid. Then the reaction liquid was extracted with chloroform. The chloroform phase was dried over magnesium sulfate, filtered and then concentrated. Then the concentrate was purified in an aluminum column to isolate 6.43 g of a greenish gray crystal.

The identification by GCMS and $^1$H-NMR revealed that the crystal was 4,5-diazafluorene.

GCMS: 168

$^1$H-NMR: ($CDCl_3$, ppm); 8.74 (d, J=4.8, 2H), 7.89 (d, J=7.8, 2H), 7.36 (dd, 2H), 3.88 (s, 2H)

A 300 mL Kjeldahl flask was charged with 6.4 g of the thus-obtained 4, 5-diazafluorene and 150 mL of dimethylformamide, and the reaction liquid was cooled to below 5°C in an ice bath. At that temperature, 4.82 g of sodium methoxide was added little by little to the reaction liquid. Subsequently 21.5 g of methyl iodide was dropwise added. After the completion of addition, the reaction liquid was stirred at room temperature for 17 hours. After the completion of reaction, 250 mL of water was added, and then the reaction liquid was extracted with chloroform. The chloroform phase was dried over anhydrous magnesium sulfate, and then filtered and concentrated. The concentrate was purified in an alumina column to give 2.84 g of a greenish purple crystal (yield: 38%).

The identification by GCMS and $^1$H-NMR revealed that the crystal was 9,9-dimethyl-4,5-diazafluorene.

GCMS: 196

$^1$H-NMR: ($CDCl_3$, ppm); 8.83 (d, 2H), 8.02 (d, 2H), 7.59 (dd, 2H), 1.61 (s, 6H)

A three-necked flask was charged with 2 g (10.2 mmol) of the thus-obtained 9,9-dimethyl-4,5-diazafluorene and 30 mL of nitrobenzene, and the content was heated to 130°C. To the content, a mixed liquid composed of 1. 6 g of bromine and 5 mL of nitrobenzene was dropwise added over a period of one hour. A reaction was carried out for 5 hours, and the reaction liquid was cooled to room temperature. An aqueous saturated sodium hydrogen carbonate solution was added to the reaction liquid to terminate the reaction. The reaction liquid was extracted with chloroform, and the chloroform phase was dried over anhydrous magnesium sulphate. Then the dried product was subjected to alumina chromatography to separate 0.59 g of the desired compound (yield: 21%) .

The identification of the compound was conducted by FDMS.

FDMS: 274

Example 1*

(Synthesis of compound 1) / * reference example

A 100 mL Kjeldahl flask equipped with a reflux condenser was charged with 2.6 g (4.4 mmol) of 2,7-dibromo-9,9'-bis[4-(methoxymethyloxy)phenyl]-9H-fluorene, prepared in Synthesis Example 1, 1.82 g (9.2 mmol) of biphenylboric acid, 14.6 g of an aqueous 20% sodium carbonate solution, 10 mg of tetrakis-(triphenylphosphine)palladium and 20 mL of tetrahydrofuran. The content was heated under reflux for 5 hours. When a predetermined time elapsed with stirring, the reaction liquid was cooled, and then an organic phase was separated. The organic phase was dried over anhydrous magnesium sulfate, and then concentrated to isolate 2.44 g of 2,7-bis(4-phenylphenyl)-9,9'-bis[4-(methoxymethyloxy)phenyl]-9H-fluorene (yield: 75%).

Then the thus-obtained 2,7-bis(4-phenylphenyl)-9,9'-bis[4-(methoxymethyloxy)phenyl]-9H-fluorene was dissolved in 20 mL of dichloromethane. To the thus-obtained solution, 5 mL (30 mmol) of an aqueous 6N-hydrochloric acid solution was added and a reaction was carried out at room temperature for 5 hours. Water was added to the reaction liquid to separate an organic phase. To the obtained organic phase, 1.03 g (13.0 mmol) of pyridine and 3.1 g (9.9 mmol) of trifluoromethanesulfonic anhydride were added, and the reaction liquid was stirred at room temperature. Water added to the reaction liquid to separate an organic phase. The organic phase was concentrated to isolate 2.7 g of the desired 2,7-bis(4-phenylphenyl)-9,9'-bis[4-(trifluoromethanesulfonyloxy)-phenyl]-9H-fluorene (yield: 90%).

This compound was identified by FDMS.

FDMS: 918

Then a 100 mL Kjeldahl flask equipped with a reflux condenser was charged with 2. 7 g of the thus-produced 2, 7-bis (4-phenylphenyl)-9,9'-bis[4-(trifluoromethanesulfonyloxy)-phenyl]-9H-fluorene, 1.2 g of biphenylboric acid, 14.6 g of an aqueous 20% sodium carbonate solution, 10 mg of tetrakis-(triphenylphosphine) palladium and 20 mL of tetrahydrofuran, and the content was heated under reflux for 4 hours. When a predetermined time elapsed with stirring, the reaction liquid was cooled to separate an organic phase. The organic phase was dried over anhydrous magnesium sulfate, and then concentrated to isolate 1.9 g of a light yellow powder. Identification by FDMS revealed that the light yellow powder was the desired 2,7-bis(4-phenylphenyl)-9,9'-bis(terphenyl-1-yl)-9H-fluorene represented by the following formula (compound 1; yield: 66%).

FDMS: 926

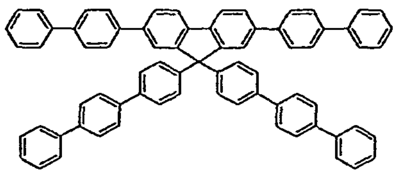

Example 2*

(Synthesis of compound 2)/ * reference example

The procedures as described in reference Example 1 were repeated wherein terphenylboric acid was used instead of the biphenylboric acid used for treating the trifluoromethanesulfonylated product with all other conditions remaining substantially the same. Thus, 2.0 g of 2,7-bis(4-phenylphenyl)-9,9'-bis(quaterphenyl-1-yl)-9H-fluorene (compound 2) represented by the following formula was isolated.

FDMS: 1078

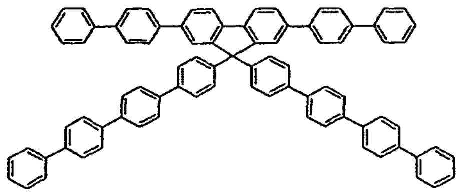

Example 3

(Synthesis of compound 24)

The procedures as described in reference Example 1 were repeated wherein 2-pyridineboric acid pinacol ester was used instead of the biphenylboric acid used for treating the trifluoromethanesulfonylated product with all other conditions remaining substantially the same. Thus, 2.0 g of 2,7-bis(4-phenylphenyl)-9,9'-bis(2-pyridylphenyl)-9H-fluorene (compound 24) represented by the following formula was isolated.

FDMS: 776

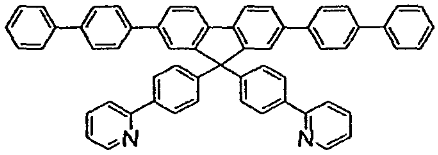

Example 4*

(Synthesis of compound 3)/ * reference example

The procedures as described in reference Example 1 were repeated wherein phenylboric acid was used instead of the biphenylboric acid used for treating the trifluoromethanesulfonylated product with all other conditions remaining substantially the same. Thus, 2.0 g of 2,7-bis(4-phenylphenyl)-9,9'-bis(4-phenylphenyl)-9H-fluorene (compound 3) represented by the following formula was isolated. The identification of compound 3 was conducted by FDMS and $^{13}C$-NMR.

The compound 3 had a melting point of 289°C and a glass transition temperature of 159°C.

Maximum fluorescence measurement on a thin film of the compound 3 revealed blue fluorescence at 417 nm. Hole mobility and electron mobility were measured at an electric field strength of about 400 $(V/cm)^{1/2}$ using a mobility analyzer available from Optel Co. by a time-of-flight method. The hole mobility was $1.5 \times 10^{-4}$ $cm^2/V$. sec, and the electron mobility was $4.0 \times 10^{-5}$ $cm^2/V$. sec. This bipolarity indicates that the compound 3 can be used as a light emitting material.

FDMS: 774

$^{13}C$-NMR: ($CDCl_3$, ppm); 152.10, 144.77, 140.60, 140.64, 140.33, 140.13, 140.05, 139.61, 139.14, 128.79, 128.68, 127.49, 127.33, 127.12, 127.00, 126.76, 124.84, 120.68, 65.32

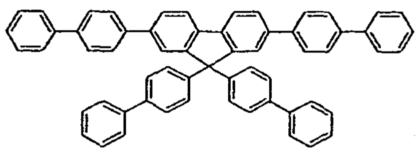

Comparative Example 1

2, 7-bis(4-phenylphenyl)-9,9'-bis [4-(methoxymethyloxy)-phenyl]-9H-fluorene prepared in reference Example 1 was dissolved in 20 mL of dichloromethane. To the thus-obtained solution, 5 mL (30 mmol) of an aqueous 6N hydrochloric acid solution was added, and a reaction was carried at room temperature for 5 hours. Water was added to the reaction liquid to separate an organic phase. The organic phase was methylated with dimethylsulfuric acid to isolate 2,7-bis(4-phenylphenyl)-9,9'-bis(4-methoxyphenyl)-9H-fluorene.

The identification of this compound was conducted by FDMS.

FDMS: 682

reference Example 5

Each (20 mg) of the compounds 1, 2 and 3, respectively prepared in reference Examples 1, 2 and 4, the compound prepared in Comparative Example 1, and Spire-6ϕ was dissolved in 2 mL of toluene to prepare a solution with a 1% concentration. A thin film was made on a quartz substrate by spin coating. The spin coating was carried out at a revolution of 1, 000 rpm for one minute, and the coating was heated at 60°C for one hour under vacuum. The thin film was allowed to leave at room temperature for one month, and its appearance was observed. Namely, it was observed whether the thin film became white, turbid or not, and whether cohesion occurred or not.

The results are shown in Table 6. As seen from Table 6, thin films of compounds 1, 2 and 3 became neither white, turbid, nor cohesion occurred.

Table 6

| Compound | Chemical formula | Appearance as observed when one month elapsed |
|---|---|---|
| Compound 1 * | 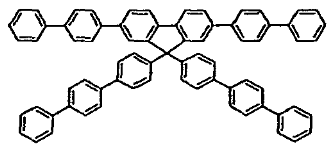 | Neitherwhite nor turbid |
| Compound 2* | 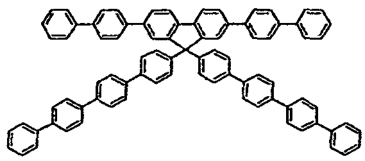 | Neitherwhite nor turbid |
| Compound 3* | 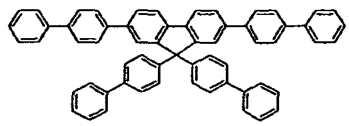 | Neitherwhite nor turbid |
| Comparative Example 1 | 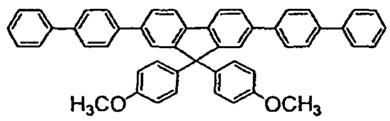 | White, turbid |
| Spiro-6ϕ | 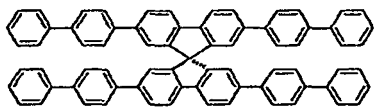 | White, turbid |

* reference example

reference Example 6

(Synthesis of compound 7)

Using 2,7 -dibromo-9,9-bis(biphenylyl)fluorene, prepared Synthesis reference example 2, and 2-bromo-9, 9-bis (biphenylyl) fluorene, and a conventional nickel catalyst, compound 7 was synthesized according to a Yamamoto coupling reaction.
FDMS: 1406

reference Example 7

(Synthesis of compound 6)

A 100 mL Kjeldahl flask was charged with 1.1 g (2.8 mmol) of 2-bromo-9, 9'-spirobifluorene, prepared in Synthesis Reference Example 4, 1.0 g (1.39 mmol) of 9,9-bis(biphenylyl)fluorene-2,7-bis(4,4,5,5-tetramethyl-[1,3,2]dioxaborolane, prepared in Synthesis Example 5, 5.5 g (11.4 mmol) of an aqueous 20% sodium carbonate solution, and 20 mL of tetrahydrofuran. Then 41 mg of bis(diphenylphosphinoferrocene) dichloropalladium as a catalyst was added to the content in a nitrogen gas atmosphere. Then the reaction liquid was heated under reflux over the night. After the reaction liquid was cooled, the reaction liquid was placed in a separatory funnel, and an organic phase was separated from the reaction liquid by the separatory funnel. The organic phase was washed with an aqueous saturated ammonium chloride solution and then with an aqueous saturated sodium chloride solution, and then concentrated to give a crystal. The crystal was purified by silica gel column chromatography using a toluene/hexane mixed solvent to give the desired compound 6
(yield: 66%).
The compound 6 had a glass transition temperature of 232°C. Maximum fluorescence measurement on a thin

film of the compound 6 revealed blue fluorescence at 421 nm.

The identification of compound 6 was conducted by FDMS, $^1$H-NMR and elementary analysis.

FDMS: 1098

$^1$H-NMR: ($CDCl_3$, ppm); 6.67-6.76 (m, 6H), 6.89 (s, 2H), 7.04-7.11 (t, 6H), 7.24-7.64 (m, 34H), 7.78-7.85 (m, 6H)

Elementary analysis: Found C: 95.2%, H: 4.8% Calculated C: 95.05%, H: 4.95%

Reference Example 8

(Synthesis of compound 5)

The procedures as described in Reference Example 7 were repeated wherein 2.8 mmol of 1-bromopyrene was used instead or 2-bromo-9,9'-spirobifluorene with all other conditions remaining substantially the same. Thus, 0.91 g of the desired compound 5 was obtained (yield: 75%).

The identification of compound 5 was conducted by FDMS and elementary analysis.

FDMS: 870

Elementary analysis: Found C: 95.2%, H: 4.8% Calculated C: 95.14%, H: 4.86%

Reference Example 9

(Synthesis of compound 12)

The procedures as described in Reference Example 7 were repeated wherein 2.8 mmol of 4,5-diaza-2'-bromo-9,9'-spirofluorene was used instead of 2-bromo-9,9'-spirobifluorene with all other conditions remaining substantially the same. Thus, 0.49 g of the desired compound 12 was obtained (yield: 32%).

The identification of compound 12 was conducted by FDMS and elementary analysis.

FDMS: 1102

Elementary analysis:

Found C: 90.3%, H: 4.6%, N: 5.1%

Calculated C: 90.35%, H: 4.57%, N: 5.08%

Reference Example 10

(Synthesis of compound 13)

The procedures as described in Reference Example 7 were repeated wherein 2.8 mmol of 2-bromo-9,9-dimethyl-4,5-diazafluorene was used instead of 2-bromo-9,9'-spirobifluorene with all other conditions remaining substantially the same. Thus, 0.49 g of the desired compound 13 was obtained (yield: 38%).

The identification of compound 13 was conducted by FDMS and elementary analysis.

FDMS: 858

Elementary analysis:

Found C: 88.1%, H: 5.4%, N: 6.5%

Calculated C: 88.08%, H: 5.4%, N: 6.52%

Reference Example 11

(Synthesis of compound 10)

The procedures as described in Reference Example 7 were repeated wherein 2.8 mmol of 5-bromo-2,3'-bipyridine was used instead of 2-bromo-9,9'-spirobifluorene with all other conditions remaining substantially the same. Thus, 0.43 g of the desired compound 10 was obtained (yield: 40%). Compound 10 had a melting point of 346°C and a glass transition temperature of 164°C.

The identification of compound 10 was conducted by FDMS and $^{13}$C-NMR.

FDMS: 778

$^{13}$C-NMR: ($CDCl_3$, ppm); 153.38, 152.54, 149.85, 148.48, 148.07, 144.23, 140.40, 139.94, 139.69, 137.23, 135.57, 135.20, 134.39, 134.16, 128.74, 128.50, 128.17, 127.23, 126.92, 124.82, 123.61, 121,25, 120.29, 65.41

Reference Example 12

(Synthesis of compound 11)

The procedures as described in Reference Example 7 were repeated wherein 2.8 mmol of 2-(4-bromophenyl)pyridine was used instead of 2-bromo-9,9'-spirobifluorene with all other conditions remaining substantially the same. Thus, 0.49 g of the desired compound 11 was obtained (yield: 45%). Compound 11 had a glass transition temperature of 174°C.

The identification of compound 11 was conducted by FDMS, $^{1}$H-NMR and $^{13}$C-NMR.
FDMS: 776
$^{1}$H-NMR: ($CDCl_3$, ppm); 7.19-7.57 (m, 22H), 7.69-7.92 (m, 12H), 8.03-8.07 (d, 4H), 8.68-8.70 (d, 2H),
$^{13}$C-NMR: ($CDCl_3$, ppm); 156.86, 152.16, 149.67, 144.74, 141.61, 140.62, 140-27, 139.67, 139.28, 138.26, 136.70, 128.70, 127.45, 127.27, 127.16, 126.98, 126.90, 124.74, 122.11, 120.75, 120.42, 65.38

Reference Example 13

(Synthesis of compound 17)

The procedures as described in Reference Example 7 were repeated wherein 2.8 mmol of 4-bromo-2,4'-bipyridine was used instead or 2-bromo-9,9'-spirobifluorene with all other conditions remaining substantially the same. Thus, 0.87 g of the desired compound 17 was obtained (yield: 80%). Compound 17 had a glass transition temperature of 176°C.

The identification of compound 17 was conducted by FDMS, $^{1}$H-NMR and elementary analysis.
FDMS: 776
$^{1}$H-NMR: ($CDCl_3$, ppm); 8.99 (s, 2H), 8.73 (d, 4H, J=6.2Hz), 7.26-8.03 (m, 32H)
Elementary analysis:

Found C: 88.2%, H: 4.7%, N: 7.1%
Calculated C: 87.9%, H: 4.9%, N: 7.2%

Reference Example 14

(Synthesis of compound 18)

A 300 mL Kjeldahl flask was charged with 60 mL of a solution in tetrahydrofuran of 2.0 g (7.3 mmol) of 3-bromo-9,9-dimethyl-4,5-diazafluorene, and the content was cooled to -78°C. While this temperature was maintained, 5 mL of a 1.6M solution of 8 mmol of n-butyllithium in hexane was dropwise added to the content, and stirred for 30 minutes. Then 2.0 g of solid dichloro-(N,N,N',N'-tetramethylethylenediamine)zinc was added to the reaction liquid, and the reaction liquid was stirred at room temperature for one hour. Then 2.2 g (3.5 mmol) of 2,6-dibromo-9,9-di(biphenylyl)fluorene and 51 mg of dichlorobis-(triphenylphosphine) palladium were added to the reaction liquid, and the mixed reaction liquid was heated under reflux over the night and then allowed to cool to room temperature. Then 30 mL of water was added to the reaction liquid, and the reaction liquid was extracted twice with toluene. An organic phase was washed with water and then dried over anhydrous magnesium sulfate, and then concentrated under a reduced pressure. The residue was purified by silica gel chromatography using a hexane/chloroform mixed liquid to give 1.02 g of a yellow crystal (yield: 34%).

The identification of the yellow crystal was conducted by FDMS.
FDMS: 858

Reference Example 15

(Synthesis of compound 19)

The procedures as described in Reference Example 14 were repeated wherein 6-bromo-2,2'-bipyridine was used instead or 2-bromo-9,9-dimethyl-4,5-diazafluorene with all other conditions remaining substantially the same. Thus, the desired compound 19 was obtained.

The identification of compound 19 was conducted by FDMS.
FDMS: 778

Reference Example 16

(Evaluation of Electron Affinity and Electron Mobility by Cyclic Voltammmetry)

The electron affinity was measured by a cell available from B.A.S. Co. under the following conditions.

Counter electrode: platinum electrode
Working electrode: glassy carbon electrode
Reference electrode: $Ag/Ag^+$
Electrolyte: tetrabutylammonium perchloride
Scanning speed: 100 mV/sec
Solvent: dichloromethane and tetrahydrofuran

Compound 12 and compound 13 exhibited an electron affinity of -2.40 eV and -2.38 eV, respectively, as ferrocene ($Fc/Fc^+$) being reference. It is to be noted that these values are comparable to or better than those (-2.41 eV) of $Alq_3$ and bathophenanthroline.

The electron mobility was measured by a time-of-flight method using a mobility analyzer available from Optel Co. Compound 12 and compound 13 exhibited an electron mobility of approximately $10^{-4}$ $cm^2/V.sec$, i.e., a higher speed than that of the conventional $Alq_3$.

Reference Example 17

(Evaluation of Electrical Stability by Cyclic Voltammetry)

Reversibility of peak was evaluated by cyclic voltammetry under the same conditions as adopted by Reference Example 16.

The results of evaluation of reversibility of peak in compound 10 as a typical example of a $\pi$-conjugated compound is shown in Fig. 1. For comparison, the results obtained with bathophenanthroline are also shown in Fig. 2.

As seen from Fig. 1 and Fig. 2, compound 10 exhibited reversibility of peak, whereas bathophenanthroline exhibited irreversibility of peak. All of the compounds as prepared in Reference Examples 1, 2, and 4 through 15 and Example 3 exhibited reversibility of peak.

Thus, the $\pi$-conjugated compound of the present invention has good electrical stability, and gives an organic EL element having enhanced durability.

Reference Example 18

(Measurement of Ionization Potential by Photoelectron Spectroscopy)

The ionization potential of compound 17 as a typical example of a $\pi$-conjugated compound was measured by "AC-3" available from Riken Keiki Co., Ltd. The ionization potential was 6.24 eV. This value is larger than those of $Alq_3$ (=5.7 eV) and CBP (=5.97 eV). Thus it has been confirmed that a $\pi$-conjugated compound can be used as a hole blocking material.

Reference Example 19

(Evaluation of EL Element)

A glass substrate provided with an ITO electrode with a thickness of 130 nm was subjected to ultrasonic cleaning using acetone and then isopropyl alcohol, and then boiling cleaning using isopropyl alcohol, and then dried. Then the cleaned glass substrate was subjected to a UV/ozone treatment to prepare a transparent electro-conductive substrate. On the ITO transparent electrode, copper phthalocyanine was vacuum-deposited to form a thin film having a thickness of 20 nm. Further, a -NPD was vacuum- deposited to form a thin film having a thickness of 40 nm on the copper phthalocyanine thin film. Thus, a hole transport layer was formed. Then aluminum trisquinolinol complex was vacuum-deposited to form a thin film having a thickness of 40 nm, and then compound 17 was vacuum- deposited thereon to form a thin film having a thickness of 20 nm. Thus an electron transport layer was formed. All of the above-mentioned vacuum deposition of the organic compounds were carried out under the same conditions of a reduced pressure of $1.0 \times 10^{-4}$ Pa and a film forming rate of 0.3 nm/sec.

LiF was vacuum-deposited to a thickness of 0.5 nm as an cathode, and aluminum was deposited thereon to a

thickness of 150 nm as a metal electrode.

On the thus-obtained assembly, a protecting glass substrate was superposed in a nitrogen gas atmosphere. The entire structure was then encapsulated with a UV curable resin.

To the thus-obtained element, direct current voltage of 6V was imposed as the ITO electrode was anode and LiF-Al electrode was cathode. The current density was 86 mA/cm$^2$, and green light emission was observed with a luminance of 3,400 cd/m$^2$.

For comparison, an element having the same layer structure as mentioned above was prepared except that the electron transport layer was formed from aluminum trisquinolinol complex ($Alq_3$). The current density was 1/2.7 of that of the above-mentioned element according to the present invention.

A relationship of current density (mA/cm$^2$) with measurement voltage (V) as obtained on the element with compound 17 and on the element with aluminum trisquinolinol complex ($Alq_3$) is shown in Fig. 3.

A relationship of luminance (cd/m$^2$) with measurement voltage (V) as obtained on the element with compound 17 and on the element with aluminum trisquinolinol complex ($Alq_3$) is shown in Fig. 4.

Luminance half life of the element with compound 17 was approximately the same as that of the element with aluminum trisquinolinol complex ($Alq_3$)

<u>Reference Example 20</u>

By the same procedures as adopted in Reference Example 19, an EL element was made and its characteristics were evaluated wherein the EL element was made using compound 19 instead of compound 17 with all other conditions and procedures remaining the same.

When a direct current voltage of 6V was imposed, the current density was 95 mA/cm$^2$, and green light emission was observed with a luminance of 3,950 cd/m$^2$.

<u>Comparative Example 2</u>

By the same procedures as adopted in Reference Example 19, an EL element was made and its characteristics were evaluated wherein the EL element was made using 4,7-diphenyl-1,10-phenanthroline (BCP) instead of compound 17 with all other conditions and procedures remaining the same.

When a direct current voltage of 6V was imposed, the current density was only 15 mA/cm$^2$, and green light emission was observed with a luminance of 510 cd/m$^2$.

Industrial Applicability

The π-conjugated compound having a cardo structure according to the present invention gives a thin film having far enhanced stability and durability, as compared with the conventional thin films. Therefore, the π-conjugated compound can be used as an organic transistor material, and a photoelectric transfer element, a solar battery and an image sensor in an organic semiconductor material, as well as a light-emitting material and an electron transport material in an organic EL element and an electrophotography photoconductor.

A π-conjugated compound as defined in claim 1. formula (1):

The π-conjugated compound has good stability and is used as light emitting material.

## Claims

**1.** A π-conjugated compound having a cardo structure represented by the following formula (1):

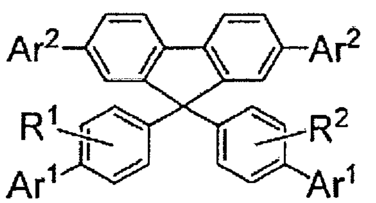

(1)

where $R^1$ and $R^2$ independently represent a hydrogen atom, a straight-chain, branched or cyclic alkyl or alkoxy group having 1 to 18 carbon atoms which may have a substituent, a phenyl, naphthyl or phenoxy group which may have a substituent, or a halogen atom;

$Ar^1$ independently represents a group represented by the following formula (3):

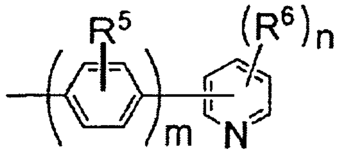

(3)

where $R^5$ and $R^6$ independently represent a hydrogen atom, a straight-chain, branched or cyclic alkyl or alkoxy group having 1 to 18 carbon atoms and m is an integer of 0 to 3, and n is an integer of 0 to 2; and

$Ar^2$ independently represents a phenyl group, a naphthyl group, an anthryl group, a phenanthryl group, a pyrenyl group, a fluorenyl group, a chrysenyl group, a perylenyl group, a picenyl group, a benzo[c]fluorenyl group, a 4-quinolyl group, a 4-pyridyl group, a 3-pyridyl group, a 2-pyridyl group, a 2-dipyridyl group, a 3-furyl group, a 2-furyl group, a 3-thienyl group, a 2-thienyl group, a 2-oxazolyl group, a 2-thiazolyl group, a 2-benzoxazolyl group, a 2-benzothiazolyl group, a 2-benzoimidazolyl group, a 1,10-phenanthrolinyl group or any one of the following formulae (4a), (4b), (5), (6a) and (6b):

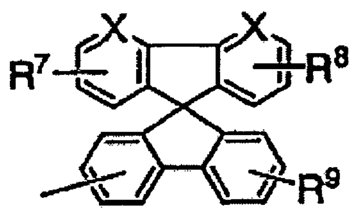

(4a)

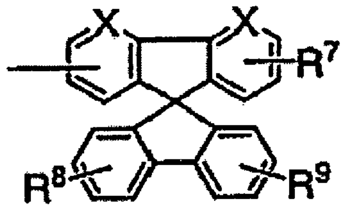

(4b)

where $R^7$, $R^3$ and $R^9$ independently represent a hydrogen atom, a straight-chain, branched or cyclic alkyl or alkoxy group having 1 to 18 carbon atoms, or an aryl or aryloxy group having 6 to 24 carbon atoms, or a heteroaryl group having 3 to 24 carbon atoms, or a halogen atom; and X represents a carbon atom or a nitrogen atom;

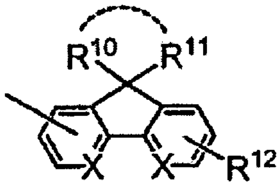

(5)

where $R^{10}$, $R^{11}$ and $R^{12}$ independently represent a hydrogen atom, a straight-chain, branched or cyclic alkyl or alkoxy group having 1 to 18 carbon atoms, or an aryl or aryloxy group having 6 to 24 carbon atoms, or a heteroaryl group having 3 to 24 carbon atoms, or a halogen atom, provided that $R^{10}$ and $R^{11}$ may be bonded together with each other to form a ring; and X represents a carbon atom or a nitrogen atom;

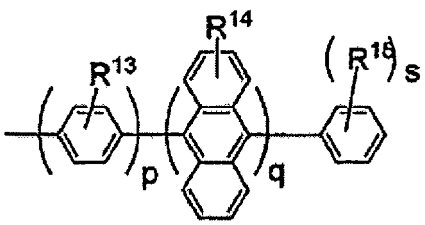

(6a)

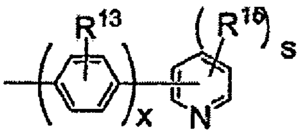

(6b)

where $R^{13}$, $R^{14}$ and $R^{15}$ independently represent a hydrogen atom, a straight-chain, branched or cyclic alkyl or alkoxy group having 1 to 10 carbon atoms, or an aryl group having 6 to 24 carbon atoms, or a heteroaryl group having 3 to 24 carbon atoms, and p and q are integers satisfying the following formula:

$$1 \leq (p + q) \leq 3$$

and s and x are integers of 0 to 2.

**2.** A process for preparing the π-conjugated compound as claimed in claim 1, which comprises the steps of:

(i) allowing a fluorene intermediate compound represented by the following formula (7):

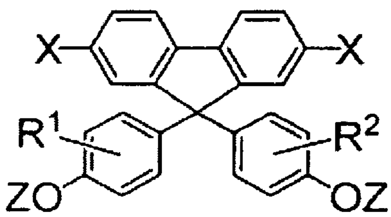

(7)

where Z is a protecting group for a phenol group, $R^1$ and $R^2$ independently represent a hydrogen atom, a straight-chain, branched or cyclic alkyl or alkoxy group having 1 to 18 carbon atoms which may have a substituent, a phenyl, naphthyl or phenoxy group which may have a substituent, or a halogen atom, and X represents an iodine atom, a bromine atom or a chlorine atom;
to react with a boric acid compound A represented by the following formula (8):

$Ar^2$–$B(OH)_2$ or 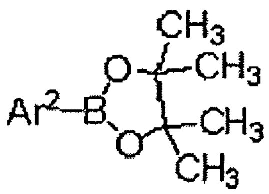 (8)

$Ar^1$–$B(OH)_2$ or 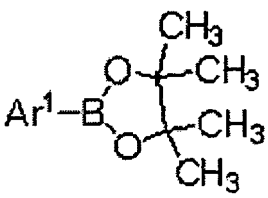 (9)

where $Ar^2$ is as defined in claim 1,
in the presence of a transition metal catalyst;
(ii) deblocking the thus-obtained reaction product in the presence of an acid catalyst;
(iii) trifluoromethanesulfonylating the deblocked product; and then,
(iv) allowing the trifluoromethanesulfonylated product to react with a boric acid compound B represented by the above formula (9),
where $Ar^1$ independently represents a group represented by the following formula (3):

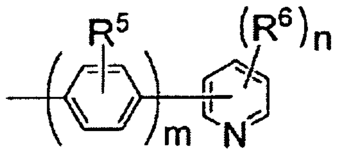

(3)

where $R^5$ and $R^6$ independently represent a hydrogen atom, a straight-chain, branched or cyclic alkyl or alkoxy group having 1 to 18 carbon atoms and m is an integer of 0 to 3, and n is an integer of 0 to 2, in the presence of a transition metal catalyst

**3.** An organic electroluminescent element characterized as having a light emission layer, a hole blocking layer or an electron transport layer, which layers comprise the π-conjugated compound as claimed in claim 1.

## Patentansprüche

**1.** π-konjugierte Verbindung mit einer durch die folgende Formel (1) dargestellten Cardo-Struktur:

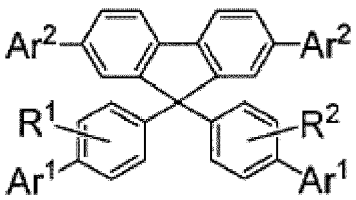

(1)

wobei $R^1$ und $R^2$ unabhängig ein Wasserstoffatom, eine geradkettige, verzweigte oder zyklische Alkyl- oder Alkoxygruppe mit 1 bis 18 Kohlenstoffatomen, welche einen Substituenten aufweisen können, eine Phenyl-, Naphthyl- oder Phenoxygruppe, welche einen Substituenten aufweisen können, oder ein Halogenatom darstellen;
$Ar^1$ unabhängig eine durch die folgende Formel (3) dargestellte Gruppe darstellt:

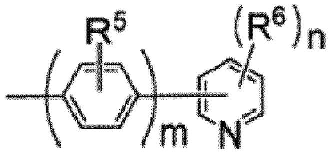

(3)

wobei $R^5$ und $R^6$ unabhängig ein Wasserstoffatom, eine geradkettige, verzweigte oder zyklische Alkyl- oder Alkoxygruppe mit 1 bis 18 Kohlenstoffatomen darstellen, und m eine ganze Zahl von 0 bis 3 ist, und n eine ganze Zahl von 0 bis 2 ist; und
$Ar^2$ unabhängig eine Phenylgruppe, eine Naphthylgruppe, eine Anthrylgruppe, eine Phenanthrylgruppe, eine Pyrenylgruppe, eine Fluorenylgruppe, eine Chrysenylgruppe, eine Perylenylgruppe, eine Picenylgruppe, eine Benzo[c]fluorenylgruppe, eine 4-Chinolylgruppe, eine 4-Pyridylgruppe, eine 3-Pyridylgruppe, eine 2-Pyridylgruppe, eine 2-Dipyridylgruppe, eine 3-Furylgruppe, eine 2-Furylgruppe, eine 3-Thienylgruppe, eine 2-Thienylgruppe, eine 2-Oxazolylgruppe, eine 2-Thiazolylgruppe, eine 2-Benzoxazolylgruppe, eine 2-Benzothiazolylgruppe, eine 2-Benzoimidazolylgruppe, eine 1,10-Phenanthrolinylgruppe oder eine der folgenden Formeln (4a), (4b), (5), (6a) und (6b) darstellt:

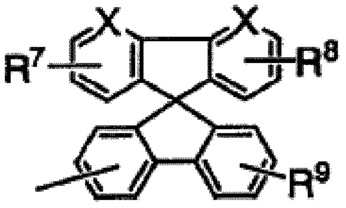

(4a)

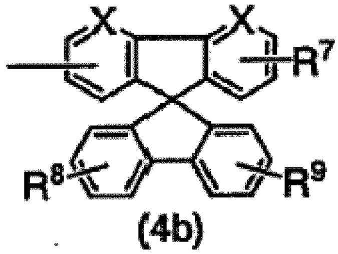

(4b)

wobei $R^7$, $R^8$ und $R^9$ unabhängig ein Wasserstoffatom, eine geradkettige, verzweigte oder zyklische Alkyl- oder Alkoxygruppe mit 1 bis 18 Kohlenstoffatomen, oder eine Aryl- oder Aryloxygruppe mit 6 bis 24 Kohlenstoffatomen, oder eine Heteroarylgruppe mit 3 bis 24 Kohlenstoffatomen, oder ein Halogenatom darstellen; und X ein Kohlenstoffatom oder ein Stickstoffatom darstellt;

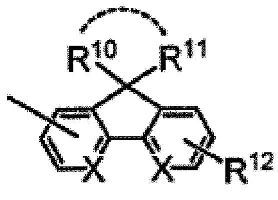

(5)

wobei $R^{10}$, $R^{11}$ und $R^{12}$ unabhängig ein Wasserstoffatom, eine geradkettige, verzweigte oder zyklische Alkyl- oder Alkoxygruppe mit 1 bis 18 Kohlenstoffatomen, oder eine Aryl- oder Aryloxygruppe mit 6 bis 24 Kohlenstoffatomen, oder eine Heteroarylgruppe mit 3 bis 24 Kohlenstoffatomen, oder ein Halogenatom darstellen, vorausgesetzt, dass $R^{10}$ und $R^{11}$ miteinander zusammenbinden können, um einen Ring zu bilden; und X ein Kohlenstoffatom oder ein Stickstoffatom darstellt;

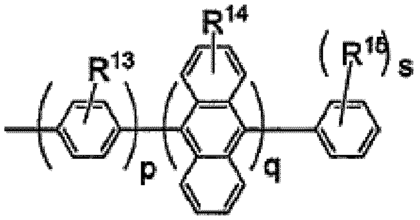

(6a)

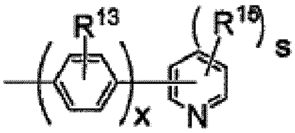

(6b)

wobei $R^{13}$, $R^{14}$ und $R^{15}$ unabhängig ein Wasserstoffatom, eine geradkettige, verzweigte oder zyklische Alkyl- oder Alkoxygruppe mit 1 bis 10 Kohlenstoffatomen, oder eine Arylgruppe mit 6 bis 24 Kohlenstoffatomen, oder eine Heteroarylgruppe mit 3 bis 24 Kohlenstoffatomen darstellen, und p und q ganze Zahlen sind, die die folgende Formel erfüllen:

$$1 \leq (p + q) \leq 3$$

und s und x ganze Zahlen von 0 bis 2 sind.

**2.** Verfahren zur Herstellung der $\pi$-konjugierten Verbindung nach Anspruch 1, welches die Schritte umfasst:

(i) Ermöglichen, dass eine durch die folgende Formel (7) dargestellte Fluoren-Intermediärverbindung:

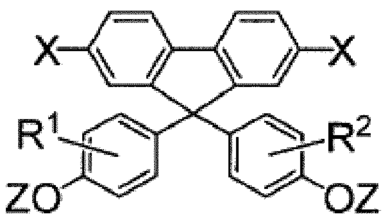

(7)

wobei Z eine Schutzgruppe für eine Phenolgruppe ist, $R^1$ und $R^2$ unabhängig ein Wasserstoffatom, eine geradkettige, verzweigte oder zyklische Alkyl- oder Alkoxygruppe mit 1 bis 18 Kohlenstoffatomen, welche einen Substituenten aufweisen können, eine Phenyl-, Naphthyl- oder Phenoxygruppe, welche einen Substituenten aufweisen können, oder ein Halogenatom darstellen, und X ein Iodatom, ein Bromatom oder ein Chloratom darstellt;
mit einer durch die folgende Formel (8) dargestellten Borsäureverbindung A reagiert:

$Ar^2-B(OH)_2$ or 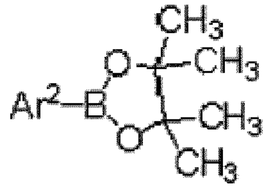 (8)

$Ar^1-B(OH)_2$ or 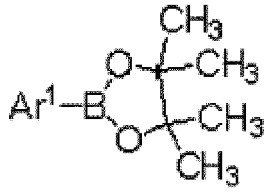 (9)

wobei $Ar^2$ wie in Anspruch 1 definiert ist,
in der Gegenwart eines Übergangsmetallkatalysators;
(ii) Entschützen des dadurch erhaltenen Reaktionsprodukts in der Gegenwart eines sauren Katalysators;
(iii) Trifluormethansulfonieren des entschützten Produkts; und anschließend,
(iv) Ermöglichen, dass das trifluormethansulfonierte Produkt mit einer durch die obige Formel (9) dargestellten Borsäureverbindung B reagiert,
wobei $Ar^1$ unabhängig eine durch die folgende Formel (3) dargestellte Gruppe darstellt:

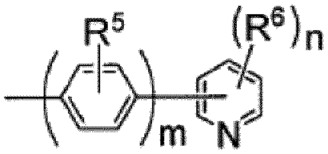 (3)

wobei $R^5$ und $R^6$ unabhängig ein Wasserstoffatom, eine geradkettige, verzweigte oder zyklische Alkyl- oder Alkoxygruppe mit 1 bis 18 Kohlenstoffatomen darstellen und m eine ganze Zahl von 0 bis 3 ist, und n eine ganze Zahl von 0 bis 2 ist, in der Gegenwart eines Übergangsmetallkatalysators.

**3.** Organisches elektrolumineszentes Element, das **gekennzeichnet ist durch** das Aufweisen einer Lichtemissionsschicht, einer lochblockierenden Schicht oder einer Elektronentransportschicht, wobei die Schichten die $\pi$-konjugierte Verbindung nach Anspruch 1 umfassen.

## Revendications

**1.** Composé $\pi$-conjugué ayant une structure de type cardo représentée par la formule (1) suivante :

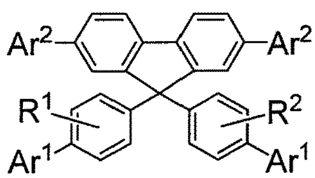 (1)

dans laquelle $R^1$ et $R^2$ représentent indépendamment un atome d'hydrogène, un groupe alkyle ou alcoxy

linéaire, ramifié ou cyclique ayant 1 à 18 atomes de carbone et pouvant porter un substituant, un groupe phényle, naphtyle ou phénoxy pouvant porter un substituant, ou un atome d'halogène ;
$Ar^1$ représente indépendamment un groupe représenté par la formule (3) suivante :

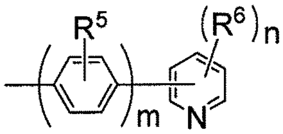
(3)

dans laquelle $R^5$ et $R^6$ représentent indépendamment un atome d'hydrogène, un groupe alkyle ou alcoxy linéaire, ramifié ou cyclique ayant 1 à 18 atomes de carbone et m est un entier de 0 à 3, et n est un entier de 0 à 2 ; et $Ar^2$ représente indépendamment un groupe phényle, un groupe naphtyle, un groupe anthryle, un groupe phénanthryle, un groupe pyrényle, un groupe fluorényle, un groupe chrysényle, un groupe pérylényle, un groupe picényle, un groupe benzo[c]fluorényle, un groupe 4-quinolyle, un groupe 4-pyridyle, un groupe 3-pyridyle, un groupe 2-pyridyle, un groupe 2-dipyridyle, un groupe 3-furyle, un groupe 2-furyle, un groupe 3-thiényle, un groupe 2-thiényle, un groupe 2-oxazolyle, un groupe 2-thiazolyle, un groupe 2-benzoxazolyle, un groupe 2-benzothiazolyle, un groupe 2-benzimidazolyle, un groupe 1,10-phénanthrolinyle ou l'une quelconque des formules (4a), (4b), (5), (6a) et (6b) suivantes :

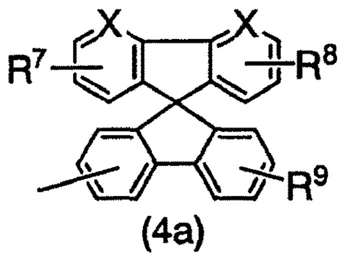
(4a)

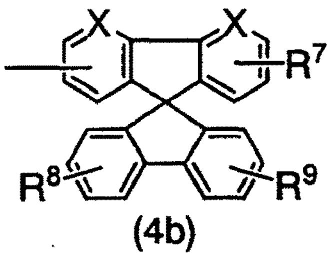
(4b)

dans lesquelles $R^7$, $R^8$ et $R^9$ représentent indépendamment un atome d'hydrogène, un groupe alkyle ou alcoxy linéaire, ramifié ou cyclique ayant 1 à 18 atomes de carbone, ou un groupe aryle ou aryloxy ayant 6 à 24 atomes de carbone, ou un groupe hétéroaryle ayant 3 à 24 atomes de carbone, ou un atome d'halogène ; et X représente un atome de carbone ou un atome d'azote ;

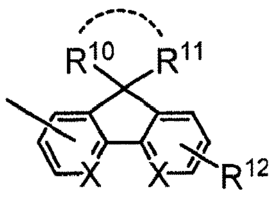
(5)

dans laquelle $R^{10}$, $R^{11}$ et $R^{12}$ représentent indépendamment un atome d'hydrogène, un groupe alkyle ou alcoxy linéaire, ramifié ou cyclique ayant 1 à 18 atomes de carbone, ou un groupe aryle ou aryloxy ayant 6 à 24 atomes de carbone, ou un groupe hétéroaryle ayant 3 à 24 atomes de carbone, ou un atome d'halogène, sous réserve que $R^{10}$ et $R^{11}$ puissent être liés ensemble l'un à l'autre pour former un cycle ; et X représente un atome de carbone ou un atome d'azote ;

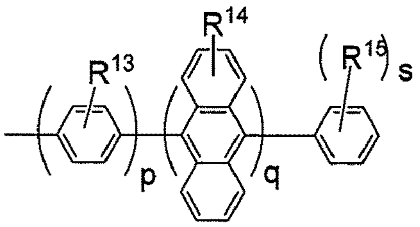

(6a)

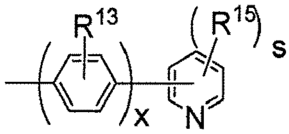

(6b)

dans lesquelles $R^{13}$, $R^{14}$ et $R^{15}$ représentent indépendamment un atome d'hydrogène, un groupe alkyle ou alcoxy linéaire, ramifié ou cyclique ayant 1 à 10 atomes de carbone, ou un groupe aryle ayant 6 à 24 atomes de carbone, ou un groupe hétéroaryle ayant 3 à 24 atomes de carbone, et p et q sont des entiers satisfaisant à la formule suivante :

$$1 \leq (p + q) \leq 3$$

et s et x sont des entiers de 0 à 2.

**2.** Procédé pour préparer le composé π-conjugué tel que revendiqué dans la revendication 1, qui comprend les étapes consistant à :

(i) laisser un composé intermédiaire fluorène représenté par la formule (7) suivante :

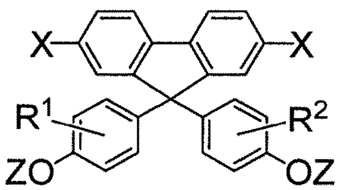

(7)

dans laquelle Z est un groupe protecteur pour un groupe phénol, $R^1$ et $R^2$ représentent indépendamment un atome d'hydrogène, un groupe alkyle ou alcoxy linéaire, ramifié ou cyclique ayant 1 à 18 atomes de carbone et pouvant porter un substituant, un groupe phényle, naphtyle ou phénoxy pouvant porter un substituant, ou un atome d'halogène, et X représente un atome d'iode, un atome de brome ou un atome de chlore ;
réagir avec un composé acide borique A représenté par la formule (8) suivante :

$Ar^2\text{-}B(OH)_2$

ou

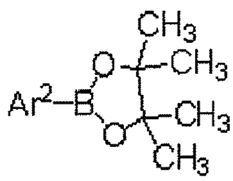

(8)

$Ar^1\text{-}B(OH)_2$

ou

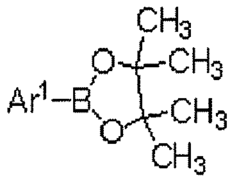

(9)

où $Ar^2$ est tel que défini dans la revendication 1,
en présence d'un catalyseur de métal de transition ;
(ii) débloquer le produit réactionnel ainsi obtenu en présence d'un catalyseur acide ;
(iii)trifluorométhanesulfonyler le produit débloqué ; et ensuite
(iv) laisser le produit trifluorométhanesulfonylé réagir avec un composé acide borique (B) représenté par la formule (9) ci-dessus,
où $Ar^1$ représente indépendamment un groupe représenté par la formule (3) suivante :

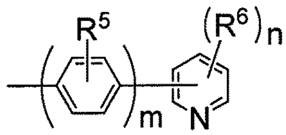

(3)

dans laquelle $R^5$ et $R^6$ représentent indépendamment un atome d'hydrogène, un groupe alkyle ou alcoxy linéaire, ramifié ou cyclique ayant 1 à 18 atomes de carbone et m est un entier de 0 à 3, et n est un entier de 0 à 2, en présence d'un catalyseur à base de métal de transition.

3. Elément électroluminescent organique **caractérisé en ce qu'**il a une couche d'émission de lumière, une couche de blocage de trous ou une couche de transport d'électrons, lesquelles couches comprennent le composé $\pi$-conjugué tel que revendiqué dans la revendication 1.

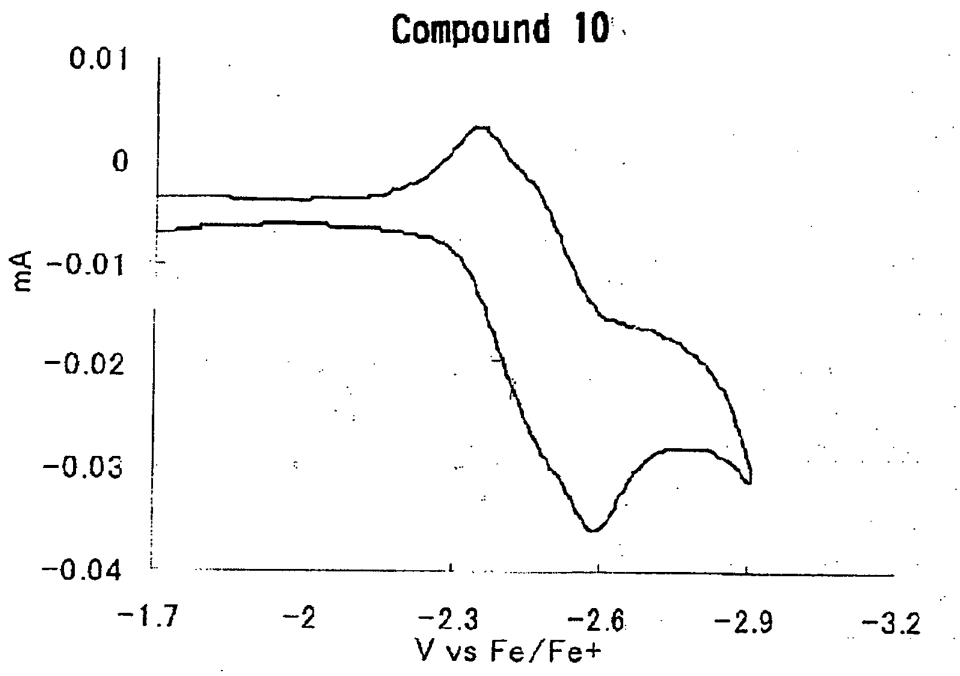
Compound 10
0.01
0
-0.01
-0.02
-0.03
-0.04
mA
-1.7
-2
-2.3
-2.6
-2.9
-3.2
V vs Fe/Fe+

FIG. 1

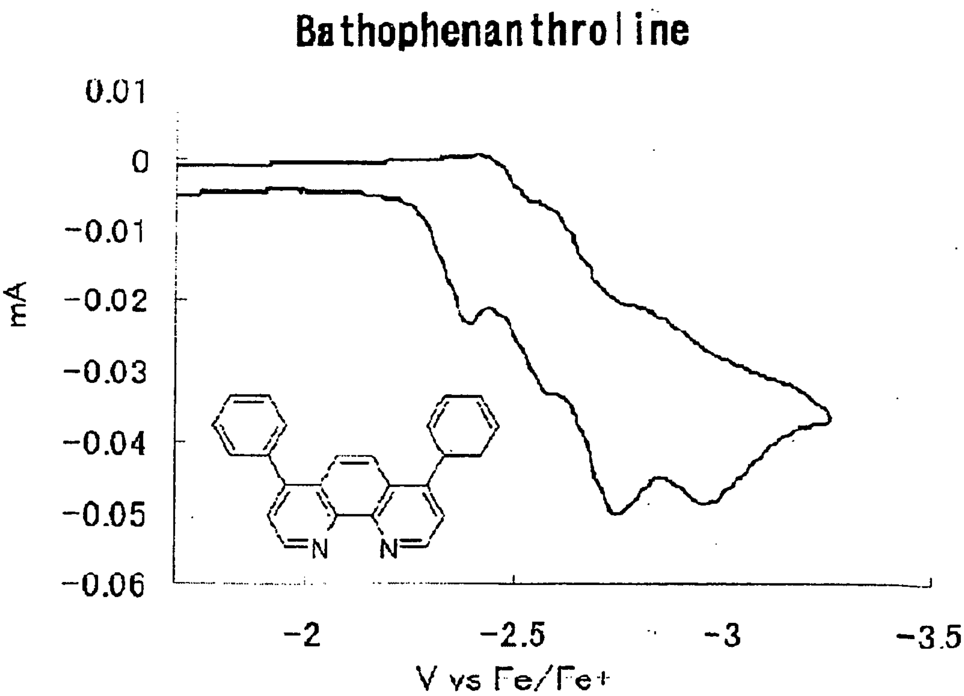
Bathophenanthroline
0.01
0
-0.01
-0.02
-0.03
-0.04
-0.05
-0.06
mA
-2
-2.5
-3
-3.5
V vs Fe/Fe+

FIG. 2

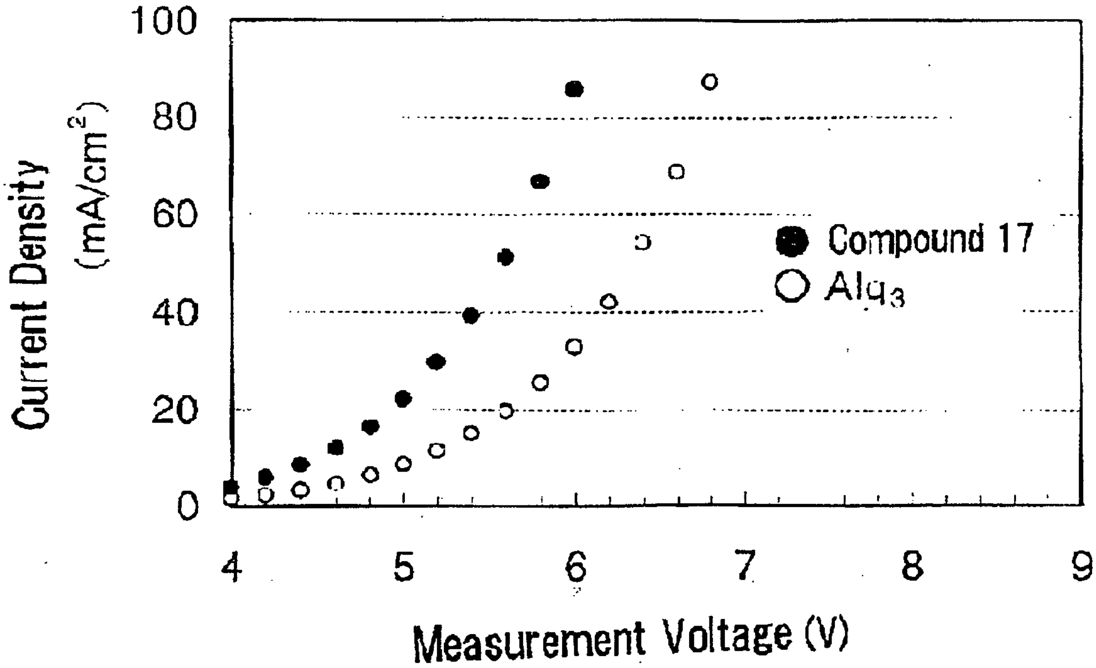
100
80
60
40
20
0
Current Density (mA/cm2)
4
5
6
7
8
9
Measurement Voltage (V)
Compound 17
Alq3

FIG. 3

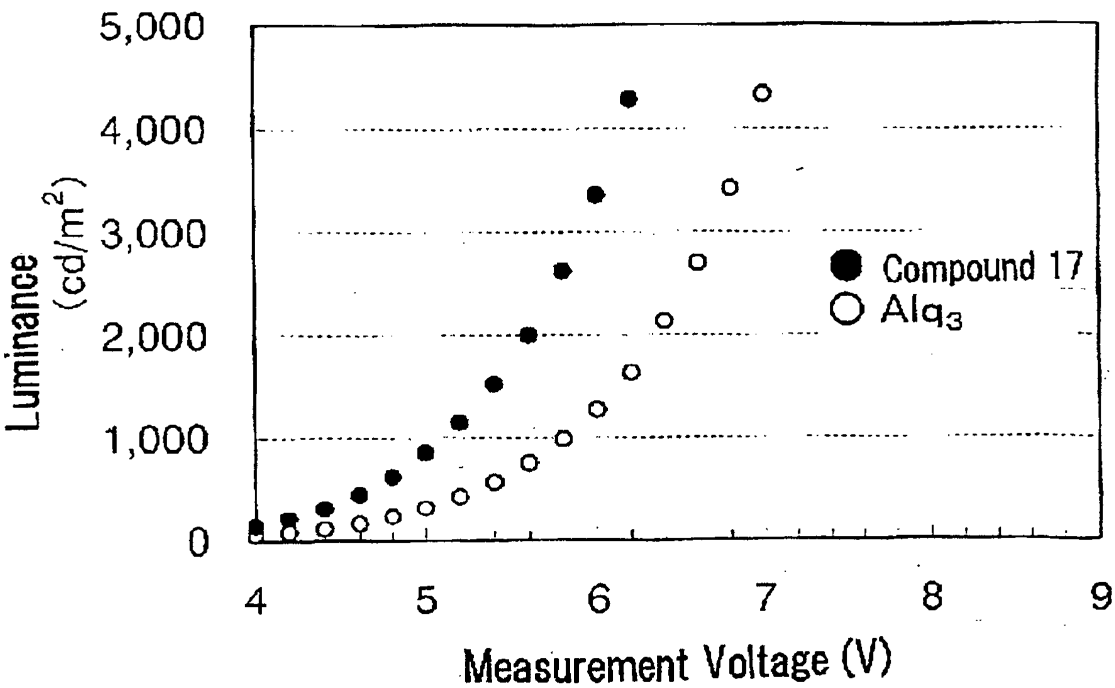
5,000
4,000
3,000
2,000
1,000
0
Luminance (cd/m2)
4
5
6
7
8
9
Measurement Voltage (V)
Compound 17
Alq3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H7278537 A
- JP 2004277377 A
- WO 2005068413 A1
- EP 1400578 A1
- EP 1400578 A
- US 2002132134 A1


### Non-patent literature cited in the description


- Organic EL Material and Display. CMC Publishing Co., Ltd, 195
- *Chem. Rev.,* vol. 19955 (95), 2457-2483
- Protecting Group in Organic Synthesis. John Wiley & Sons